# EUROPÄISCHE PATENTSCHRIFT

(11) **EP 2 245 001 B1**
(45) Veröffentlichungstag und Bekanntmachung des Hinweises auf die Patenterteilung: **20.03.2013**
(21) Anmeldenummer: 08866428.9
(22) Anmeldetag: 16.12.2008
(51) Int. Cl.: C07C 227/16, C07C 229/30

(54) **Verfahren zum Herstellen von 2-Fluoracyl-3-amino-acrylsäure-derivaten**
Method for the production of 2-fluoroacyl-3-amino-acrylic acid derivatives
Procédé de fabrication de dérivés d'acide acrylique 2-fluoracyle-3-amino

(30) Priorität: 21.12.2007 EP 07150357
(43) Veröffentlichungstag der Anmeldung: 03.11.2010
(73) Patentinhaber: Bayer CropScience AG, 40789 Monheim (DE)
(72) Erfinder: LUI, Norbert, 51519 Odenthal (DE); WOLLNER, Thomas, 50825 Köln (DE); WIESCHEMEYER, Jürgen, 51467 Bergisch Gladbach (DE); DOCKNER, Michael, 50674 Köln (DE); HEINRICH, Jens-Dietmar, 51399 Burscheid (DE)
(74) Vertreter: Bader, Axel Jochen
(86) Internationale Anmeldenummer: PCT/EP2008/010689
(87) Internationale Veröffentlichungsnummer: WO 2009/083134

(56) Entgegenhaltungen:
- WO-A-03/051820
- WO-A-2005/042468
- US-A1- 2007 225 280
- HOJO M ET AL: "ELECTROPHILIC SUBSTITUTIONS OF OLEFINIC HYDROGENS II. ACYLATION OF VINYL ETHERS AND N-VINYL AMIDES" CHEMISTRY LETTERS, CHEMICAL SOCIETY OF JAPAN. TOKYO, 1. Januar 1976 (1976-01-01), Seiten 499-502, XP000196322 ISSN: 0366-7022

## Beschreibung

Die vorliegende Erfindung betrifft ein Verfahren zum Herstellen von 2-Fluoracyl-3-amino-acrylsäure-Derivaten durch Umsetzung von fluorierten Carbonsäuren mit Dialkylaminoacrylsäure-Derivaten und Säurehalogeniden.

2-Fluoracyl-3-amino-acrylsäure-Derivate wie z.B. 2-Trifluormethyl-3-aminoacrylsäureester und 2-Difluormethyl-3-aminoacrylsäureester sind wertvolle Zwischenprodukte zur Herstellung von substituierten Pyrazolen, die als Fungizide Verwendung finden können.

Es ist bereits bekannt, dass man trihalogenacylierte Aminoacrylsäureester erhält, wenn man die entsprechenden Chloracroleine mit einem substituierten Amin umsetzt (vgl. Tetrahedron Lett. 1996, 37, 8751 - 8754). Nachteilig an diesem Verfahren ist jedoch, dass die als Ausgangsverbindungen eingesetzten Chloracroleine schwierig herzustellen und für eine technische Anwendung zu teuer sind.

Aus EP-A-1 000 926 ist bekannt, dass man Trihaloacyl-aminopropenoate erhält, indem man Trihalogenacetoacetate mit Dialkylformamidacetalen umsetzt. Nachteilig ist hier, dass als Nebenprodukt die deacylierten Verbindungen auftreten und vom gewünschten Produkt abgetrennt werden müssen.

WO-A-03/051820 offenbart, dass man 2-Perhalogenacyl-3-amino-acrylsäure-Derivate erhalten kann, indem man 3-Aminoacrylsäureester mit Perhalogenalkylcarbonsäureanhydriden umsetzt. Nachteilig ist hier, dass man zur Herstellung der fluorierte Derivate niedrig siedende und teure Säurechloride bzw. Anhydride benötigt. Beim Einsatz von z.B. Trifluoressigsäureanhydrid verliert man ein Äquivalent des wertvollen fluorierten Bausteins.

WO-A-05/042468 lehrt ein Verfahren zum Herstellen von 2-Dihalogenacyl-3-amino-acrylsäureestem bei dem Säurehalogenide eingesetzt werden. Diese Säurechloride sind aufwendig aus den Säuren herzustellen. Außerdem ist ihr Einsatz aufgrund der niedrigen Siedepunkte schwierig.

Hojo et al., Chemistry Letters, Chemical Society of Japan: 499-502 (1976) beschreibt die Reaktion von Vinyl-Ethern, Vinyl-Sulfiden und Vinyl-Amiden mit gemischten Anhydriden der Formel CH₃COOCOCF₃, die zu den trifluoracetylierten (anstelle der acetylierten) Verbindungen reagieren_{.} Da in dem erfindungsgemäßen Verfahren keine Säureanhydride als Edukte eingesetzt werden und in situ auch keine Halogenalkyl-substituierten gemischen Anhydride nachgewiesen werden können, stellt die Publikation von Hojo keinen Ausgangspunkt für das erfindungsgemäße Verfahren dar.
Die Aufgabe der vorliegenden Erfindung bestand in der Bereitstellung neuer, wirtschaftlicher Verfahren zum Herstellen von 2-Fluoracyl-3-amino-acrylsäure-Derivaten ausgehend von fluorierten Carbonsäuren, die die zuvor beschriebenen Nachteile nicht aufweisen.

Die Aufgabe wurde gemäß der vorliegenden Erfindung gelöst durch ein Verfahren zum Herstellen von 2-Fluoracyl-3-amino-acrylsäure-Derivaten der allgemeinen Formel (I) 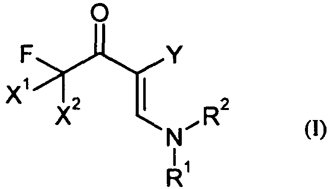 in welcher
- R¹ und R²: unabhängig voneinander ausgewählt sind aus C₁₋₁₂-Alkyl-, C₃₋₋₈-Cycloalkyl-, C₂₋₁₂-Alkenyl-, C₂₋₁₂-Alkinyl-, C₆₋₈-Aryl-, C₇₋₁₉-Arylalkyl- oder C₇₋₁₉-Alkylaryl-Gruppen, die jeweils mit einer oder mehreren Gruppen, die ausgewählt sind aus der Gruppe bestehend aus -R', -X, -OR', -SR', -NR'₂, -SiR'₃, -COOR', -(C=O)R', -CN and-CONR₂', wobei R' Wasserstoff oder eine C₁₋₁₂-Alkyl-Gruppe ist, substituiert sein können;
- R¹ und R²: zusammen mit dem Stickstoffatom, an das sie gebunden sind, einen 5- bis 6-gliedrigen Ring bilden können, der gegebenenfalls ein oder zwei weitere Hetero-Atome, ausgewählt aus O, S und einer SO₂-Gruppe, enthalten kann und der mit einer oder mehreren Gruppen, die ausgewählt sind aus der Gruppe bestehend aus - R', -X, -OR', -SR', -NR'₂, -SiR'₃, -COOR', -(C=O)R', -CN and -CONR₂', wobei R' Wasserstoff oder eine C₁₋₁₂-Alkyl-Gruppe ist, substituiert sein kann;
- Y: ausgewählt ist aus (C=O)OR³, CN und (C=O)NR⁴R⁵, wobei R³ ,R⁴ und R⁵ unabhängig von einander ausgewählt sind aus C₁₋₁₂-Alkyl-, C₃₋₈-Cycloalkyl, C₂₋₁₂-Alkenyl-, C₂₋₁₂-Alkinyl-, C₆₋₈-Aryl-, C₇₋₁₉-Arylalkyl- oder C₇₋₁₉-Alkylaryl-Gruppen, die jeweils mit einer oder mehreren Gruppen, die ausgewählt sind aus der Gruppe bestehend aus -R', -X, -OR', -SR', -NR'₂, -SiR'₃, -COOR', -(C=O)R', -CN and-CONR₂', wobei R' Wasserstoff oder eine C₁₋₁₂-Alkyl-Gruppe ist, substituiert sein können;
und
- X¹ und X²: unabhängig voneinander für Fluor, Chlor, Brom, Wasserstoff, C₁₋₁₂-Alkyl-, C₃₋₈-Cycloalkyl-, C₂₋₁₂-Alkenyl-, C₂₋₁₂-Alkinyl-, C₆₋₈-Aryl-, C₇₋₁₉-Arylalkyl- oder C₇₋₁₉-Alkylaryl-Gruppen stehen, wobei die zuvor genannten Alkyl-, Cycloalkyl-, Alkenyl-, Alkinyl-, Aryl-, Arylalkyl- oder Alkylaryl-Gruppen jeweils mit einer oder mehreren Gruppen, die ausgewählt sind aus der Gruppe bestehend aus -R', -X, -OR', -SR', -NR'₂, -SiR'₃, -COOR', -(C=O)R', -CN and -CONR₂', wobei R' Wasserstoff oder eine C₁₋₁₂-Alkyl-Gruppe ist, substituiert sein können;
dadurch gekennzeichnet, dass man
fluorierte Carbonsäuren der Formel (II) 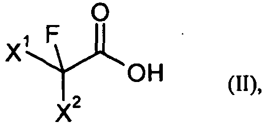 in welcher X¹ und X² die oben genannte Bedeutungen haben,
mit 3-Amino-acrylsäure-Derivaten der Formel (III) 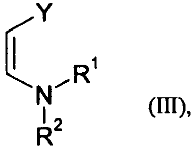 in welcher R¹, R² und Y die oben genannte Bedeutungen haben,
und, in Gegenwart einer Base, mit einem Säurehalogenid der Formel (IV) 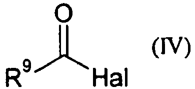 in welcher
- R⁹: ausgewählt ist aus C₁₋₁₂-Alkyl-, C₃₋₈-Cycloalkyl-, C₂₋₁₂-Alkenyl-, C₂₋₁₂-Alkinyl-, C₆₋₈-Aryl-, C₇₋₁₉-Arylalkyl- oder C₇₋₁₉-Alkylaryl-Gruppen, die jeweils mit einer oder mehreren Gruppen, die ausgewählt sind aus der Gruppe bestehend aus -R', -X, -OR', -SR', -NR'₂, -SiR'₃, -COOR', -(C=O)R', -CN and -CONR₂', wobei R' Wasserstoff oder eine C₁₋₁₂-Alkyl-Gruppe ist, substituiert sein können;
- Hal: für Halogen steht; umsetzt.

Überraschenderweise lassen sich die 2-Fluoracyl-3-amino-acrylsäure-Derivate der Formel (I) unter den erfindungsgemäßen Bedingungen mit guten Ausbeuten in hoher Reinheit herstellen, womit das erfindungsgemäße Verfahren die im Zusammenhang mit dem Stand der Technik beschriebenen Nachteile nicht aufweist.

Das erfindungsgemäße Verfahren kann anhand des folgenden Schema (I) erläutert werden :

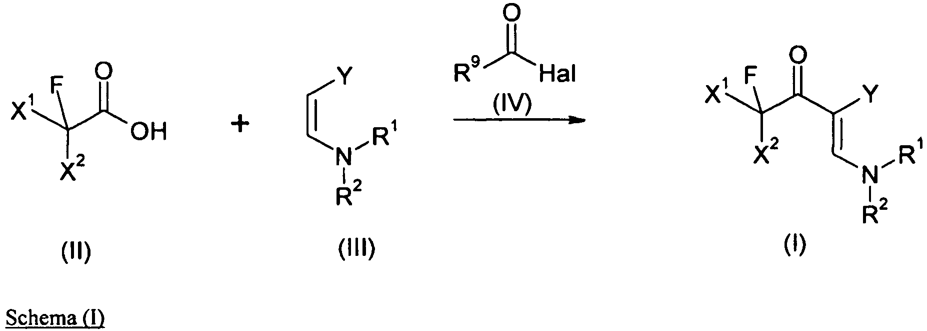

### Allgemeine Definitionen

Im Zusammenhang mit der vorliegenden Erfindung umfasst der Begriff Halogene (X), soweit nicht anders definiert, solche Elemente, die ausgewählt sind aus der Gruppe bestehend aus Fluor, Chlor, Brom und Iod, wobei Fluor, Chlor und Brom bevorzugt und Fluor und Chlor besonders bevorzugt verwendet werden.

Gegebenenfalls substituierte Gruppen können einfach oder mehrfach substituiert sein, wobei bei Mehrfachsubstitutionen die Substituenten gleich oder verschieden sein können.

Mit einem oder mehreren Halogenatomen (-X) substituierte Alkyl-Gruppen sind beispielsweise ausgewählt aus Trifluormethyl (CF₃), Difluormethyl (CHF₂), CF₃CH₂, ClCH₂, CF₃CCl₂.

Alkyl-Gruppen sind im Zusammenhang mit der vorliegenden Erfindung, soweit nicht abweichend definiert, lineare oder verzweigte Kohlenwasserstoff-Gruppen, die optional ein, zwei oder mehrere Heteroatome, die ausgewählt sind aus O, N, P und S aufweisen können. Außerdem können die erfindungsgemäßen Alkyl-Gruppen optional durch weitere Gruppen substituiert sein, die ausgewählt sind aus -R', Halogen- (-X), Alkoxy- (-OR'), Thioether- oder Mercapto- (-SR'), Amino- (-NR'₂), Silyl- (-SiR'₃), Carboxyl- (-COOR'), Cyan- (-CN), Acyl- (-(C=O)R') und Amid-Gruppen (-CONR₂'), wobei R' Wasserstoff oder eine C₁₋₁₂-Alkyl-Gruppe, vorzugsweise C₂₋₁₀-Alkyl-Gruppe, besonders bevorzugt C₃₋₈-Alkyl-Gruppe ist, die ein oder mehrere Heteroatome, ausgewählt aus N, O, P und S, aufweisen kann.

Cycloalkyl-Gruppen sind im Zusammenhang mit der vorliegenden Erfindung, soweit nicht abweichend definiert, monocyclische, gesättigte Kohlenwasserstoffgruppen mit 3 bis 8 Kohlenstoffringgliedern, die optional ein, zwei oder mehrere Heteroatome, die ausgewählt sind aus O, N, P und S aufweisen können. Außerdem können die erfindungsgemäßen Cycloalkyl-Gruppen optional durch weitere Gruppen substituiert sein, die ausgewählt sind aus -R', Halogen- (-X), Alkoxy- (-OR'), Thioether- oder Mercapto- (-SR'), Amino- (-NR'₂), Silyl- (-SiR'₃), Carboxyl- (-COOR'), Cyan-(-CN), Acyl- (-(C=O)R') und Amid-Gruppen (-CONR₂'), wobei R' Wasserstoff oder eine C₁₋₁₂-Alkyl-Gruppe, vorzugsweise C₂₋₁₀-Alkyl-Gruppe, besonders bevorzugt C₃₋₈-Alkyl-Gruppe ist, die ein oder mehrere Heteroatome, ausgewählt aus N, O, P und S, aufweisen kann.

Im Einzelnen umfasst diese Definition beispielsweise die Bedeutungen Cyclopropyl, Cyclobutyl, Cyclopentyl und Cyclohexyl.

Die Definition C₁-C₁₂-Alkyl umfasst den größten hierin definierten Bereich für einen Alkyl-Gruppe. Im Einzelnen umfasst diese Definition beispielsweise die Bedeutungen Methyl, Ethyl, n-, iso-Propyl, n-, iso-, sec- und t-Butyl, n-Pentyl, n-Hexyl, 1,3-Dimethylbutyl, 3,3-Dimethylbutyl, n-Heptyl, n-Nonyl, n-Decyl, n-Undecyl, n-Dodecyl.

Alkenyl-Gruppen sind im Zusammenhang mit der vorliegenden Erfindung, soweit nicht abweichend definiert, lineare oder verzweigte Kohlenwasserstoff-Gruppen, die wenigstens eine einfache Unsättigung (Doppelbindung) enthalten und optional eine, zwei oder mehrere einfache oder zweifache Unsättigungen oder ein, zwei oder mehrere Heteroatome, die ausgewählt sind aus O, N, P und S aufweisen können. Außerdem können die erfindungsgemäßen Alkenyl-Gruppen optional durch weitere Gruppen substituiert sein, die ausgewählt sind aus -R', Halogen- (-X), Alkoxy- (-OR'), Thioether- oder Mercapto- (-SR'), Amino- (-NR'₂), Silyl- (-SiR'₃), Carboxyl- (-COOR'), Cyan-(-CN), Acyl- (-(C=O)R') und Amid-Gruppen (-CONR₂'), wobei R' Wasserstoff oder eine C₁₋₁₂-Alkyl-Gruppe, vorzugsweise C₂₋₁₀-Alkyl-Gruppe, besonders bevorzugt C₃₋₈-Alkyl-Gruppe ist, die ein oder mehrere Heteroatome, ausgewählt aus N, O, P und S, aufweisen kann.

Die Definition C₂-C₁₂-Alkenyl umfasst den größten hierin definierten Bereich für einen AlkenylGruppe. Im Einzelnen umfasst diese Definition beispielsweise die Bedeutungen Vinyl; Allyl (2-Propenyl), Isopropenyl (1-Methylethenyl); But-1-enyl (Crotyl), But-2-enyl, But-3-enyl; Hex-1-enyl, Hex-2-enyl, Hex-3-enyl, Hex-4-enyl, Hex-5-enyl; Hept-1-enyl, Hept-2-enyl, Hept-3-enyl, Hept-4-enyl, Hept-5-enyl, Hept-6-enyl; Oct-1-enyl, Oct-2-enyl, Oct-3-enyl, Oct-4-enyl, Oct-5-enyl, Oct-6-enyl, Oct-7-enyl; Non-1-enyl, Non-2-enyl, Non-3-enyl, Non-4-enyl, Non-5-enyl, Non-6-enyl, Non-7-enyl, Non-8-enyl; Dec-1-enyl, Dec-2-enyl, Dec-3-enyl, Dec-4-enyl, Dec-5-enyl, Dec-6-enyl, Dec-7-enyl, Dec-8-enyl, Dec-9-enyl; Undec-1-enyl, Undec-2-enyl, Undec-3-enyl, Undec-4-enyl, Undec-5-enyl, Undec-6-enyl, Undec-7-enyl, Undec-8-enyl, Undec-9-enyl, Undec-10-enyl; Dodec-1-enyl, Dodec-2-enyl, Dodec-3-enyl, Dodec-4-enyl, Dodec-5-enyl, Dodec-6-enyl, Dodec-7-enyl, Dodec-8-enyl, Dodec-9-enyl, Dodec-10-enyl, Dodec-11-enyl; Buta-1,3-dienyl, Penta-1,3-dienyl.

Cycloalkenyl-Gruppen sind im Zusammenhang mit der vorliegenden Erfindung, soweit nicht abweichend definiert, monocyclische, nicht aromatische Kohlenwasserstoffgruppen mit 3 bis 8 Kohlenstoffringgliedem mit mindestens einer Doppelbindung, die optional ein, zwei oder mehrere Heteroatome, die ausgewählt sind aus O, N, P und S aufweisen können. Außerdem können die erfindungsgemäßen Cycloalkenyl-Gruppen optional durch weitere Gruppen substituiert sein, die ausgewählt sind aus -R', Halogen- (-X), Alkoxy- (-OR'), Thioether- oder Mercapto- (-SR'), Amino-(-NR'₂), Silyl- (-SiR'₃), Carboxyl- (-COOR'), Cyan- (-CN), Acyl- (-(C=O)R') und Amid-Gruppen (-CONR₂'), wobei R' Wasserstoff oder eine C₁₋₁₂-Alkyl-Gruppe, vorzugsweise C₂₋₁₀-Alkyl-Gruppe, besonders bevorzugt C₃₋₈-Alkyl-Gruppe ist, die ein oder mehrere Heteroatome, ausgewählt aus N, O, P und S, aufweisen kann.

Im Einzelnen umfasst diese Definition beispielsweise die Bedeutungen Cyclopenten-1-yl, Cyclohexen-1-yl, Cyclohepta-1,3-dien-1-yl.

Alkinyl-Gruppen sind im Zusammenhang mit der vorliegenden Erfindung, soweit nicht abweichend definiert, lineare, verzweigte oder ringförmige Kohlenwasserstoff-Gruppen, die wenigstens eine zweifache Unsättigung (Dreifachbindung) enthalten und optional eine, zwei oder mehrere einfache oder zweifache Unsättigungen oder ein, zwei oder mehrere Heteroatome, die ausgewählt sind aus O, N, P und S aufweisen können. Außerdem können die erfindungsgemäßen Alkinyl-Gruppen optional durch weitere Gruppen substituiert sein, die ausgewählt sind aus -R', Halogen- (-X), Alkoxy- (-OR'), Thioether- oder Mercapto- (-SR'), Amino- (-NR'₂), Silyl- (-SiR'₃), Carboxyl- (-COOR'), Cyan-(-CN), Acyl- (-(C=0)R') und Amid-Gruppen (-CONR₂'), wobei R' Wasserstoff oder eine lineare, verzweigte oder cyclische C₁₋₁₂-Alkyl-Gruppe ist, die ein oder mehrere Heteroatome, ausgewählt aus N, O, P und S, aufweisen kann.

Die Definition C₂-C₁₂-Alkinyl umfasst den größten hierin definierten Bereich für einen Alkinyl-Gruppe. Im Einzelnen umfasst diese Definition beispielsweise die Bedeutungen Ethinyl (Acetylenyl); Prop-1-inyl und Prop-2-inyl.

Aryl-Gruppen sind im Zusammenhang mit der vorliegenden Erfindung, soweit nicht abweichend definiert, aromatische Kohlenwasserstoff-Gruppen, die ein, zwei oder mehrere Heteroatome, die ausgewählt sind aus O, N, P und S aufweisen können und optional durch weitere Gruppen substituiert sein können, die ausgewählt sind aus -R', Halogen- (-X), Alkoxy- (-OR'), Thioether- oder Mercapto-(-SR'), Amino- (-NR'₂), Silyl- (-SiR'₃), Carboxyl- (-COOR'), Cyan- (-CN), Acyl- (-(C=O)R') und Amid-Gruppen (-CONR₂'), wobei R' Wasserstoff oder eine C₁₋₁₂-Alkyl-Gruppe, vorzugsweise C₂₋₁₀-Alkyl-Gruppe, besonders bevorzugt C₃₋₈-Alkyl-Gruppe ist, die ein oder mehrere Heteroatome, ausgewählt aus N, O, P und S, aufweisen kann.

Die Definition C₅₋₁₈-Aryl umfasst den größten hierin definierten Bereich für eine Aryl-Gruppe mit 5 bis 18 Gerüst-Atomen, wobei die C-Atome gegen Heteroatome ausgetauscht sein können. Im Einzelnen umfasst diese Definition beispielsweise die Bedeutungen Cyclopentadienyl, Phenyl, Cycloheptatrienyl, Cyclooctatetraenyl, Naphthyl und Anthracenyl; 2-Furyl, 3-Furyl, 2-Thienyl, 3-Thienyl, 2-Pyrrolyl, 3-Pyrrolyl, 3-Isoxazolyl, 4-Isoxazolyl, 5-Isoxazolyl, 3-Isothiazolyl, 4-Isothiazolyl, 5-Isothiazolyl, 3-Pyrazolyl, 4-Pyrazolyl, 5-Pyrazolyl, 2-Oxazolyl, 4-Oxazolyl, 5- Oxazolyl, 2-Thiazolyl, 4-Thiazolyl, 5-Thiazolyl, 2-Imidazolyl, 4-Imidazolyl, 1,2,4-Oxadiazol-3-yl, 1,2,4-Oxadiazol-5-yl, 1,2,4-Thiadiazol-3-yl, 1,2,4-Thiadiazol-5-yl, 1,2,4-Triazol-3-yl, 1,3,4-Oxadiazol-2-yl, 1,3,4-Thiadiazol-2-yl und 1,3,4-Triazol-2-yl; 1-Pyrrolyl, 1-Pyrazolyl, 1,2,4-Triazol-1-yl, 1-Imidazolyl, 1,2,3-Triazol-1-yl, 1,3,4-Triazol-1-yl; 3-Pyridazinyl, 4-Pyridazinyl, 2-Pyrimidinyl, 4-Pyrimidinyl, 5-Pyrimidinyl, 2-Pyrazinyl, 1,3,5-Triazin-2-yl und 1,2,4-Triazin-3-yl.

Arylalkyl-Gruppen (Aralkyl-Gruppen) sind im Zusammenhang mit der vorliegenden Erfindung, soweit nicht abweichend definiert, durch Aryl-Gruppen substituierte Alkyl-Gruppen, die eine C₁₋₈-Alkylenkette aufweisen können und im Arylgerüst oder der Alkylenkette durch eines oder mehrere Heteroatome, die ausgewählt sind aus O, N, P und S und optional durch weitere Gruppen substituiert sein können, die ausgewählt sind aus -R', Halogen- (-X), Alkoxy- (-OR'), Thioether- oder Mercapto-(-SR'), Amino- (-NR'₂), Silyl- (-SiR'₃), Carboxyl- (-COOR'), Cyan- (-CN), Acyl- (-(C=O)R') und Amid-Gruppen (-CONR₂'), wobei R' Wasserstoff oder eine C₁₋₁₂-Alkyl-Gruppe, vorzugsweise C₂₋₁₀-Alkyl-Gruppe, besonders bevorzugt C₃₋₈-Alkyl-Gruppe ist, die ein oder mehrere Heteroatome, ausgewählt aus N, O, P und S, aufweisen kann.

Die Definition C₇₋₁₉-Aralkyl-Gruppe umfasst den größten hierin definierten Bereich für einen Arylalkyl-Gruppe mit insgesamt 7 bis 19 Atomen in Gerüst und Alkylenkette. Im Einzelnen umfasst diese Definition beispielsweise die Bedeutungen Benzyl- und Phenylethyl-.

Alkylaryl-Gruppen (Alkaryl-Gruppen) sind im Zusammenhang mit der vorliegenden Erfindung, soweit nicht abweichend definiert, durch Alkyl-Gruppen substituierte Aryl-Gruppen, die eine C₁₋₈-Alkylenkette aufweisen können und im Arylgerüst oder der Alkylenkette durch eines oder mehrere Heteroatome, die ausgewählt sind aus O, N, P und S und optional durch weitere Gruppen substituiert sein können, die ausgewählt sind aus -R', Halogen- (-X), Alkoxy- (-OR'), Thioether- oder Mercapto-(-SR'), Amino- (-NR'₂), Silyl- (-SiR'₃), Carboxyl- (-COOR'), Cyan- (-CN), Acyl- (-(C=O)R') und Amid-Gruppen (-CONR₂'), wobei R' Wasserstoff oder eine C₁₋₁₂-Alkyl-Gruppe, vorzugsweise C₂₋₁₀-Alkyl-Gruppe, besonders bevorzugt C₃₋₈-Alkyl-Gruppe ist, die ein oder mehrere Heteroatome, ausgewählt aus N, O, P und S, aufweisen kann.

Die Definition C₇₋₁₉-Alkylaryl-Gruppe umfasst den größten hierin definierten Bereich für einen Alkylaryl-Gruppe mit insgesamt 7 bis 19 Atomen in Gerüst und Alkylenkette. Im Einzelnen umfasst diese Definition beispielsweise die Bedeutungen Tolyl-, 2,3-, 2,4-, 2,5-, 2,6-, 3,4- oder 3,5-Dimethylphenyl.

Die Alkyl-, Alkenyl-, Alkinyl-, Aryl,- Alkaryl- und Aralkylgruppen können zudem ein oder mehrere Heteroatome aufweisen, die - soweit nicht abweichend definiert - ausgewählt sind aus N, O, P und S. Die Heteroatome ersetzen dabei die bezifferten Kohlenstoffatome.

Die erfindungsgemäßen Verbindungen können gegebenenfalls als Mischungen verschiedener möglicher isomerer Formen, insbesondere von Stereoisomeren, wie z.B. E- und Z-, threo- und erythro-, sowie optischen Isomeren, gegebenenfalls aber auch von Tautomeren vorliegen. Es werden sowohl die E- als auch die Z-Isomeren, wie auch die threo- und erythro-, sowie die optischen Isomeren, beliebige Mischungen dieser Isomeren, sowie die möglichen tautomeren Formen offenbart und beansprucht.

### Dialkylaminoacrylsäur-Derivate (III)

Die bei der Durchführung des erfindungsgemäßen Verfahrens als Ausgangsstoffe verwendeten Dialkylaminoacrylsäure-Derivate sind durch die Formel (III) allgemein definiert.

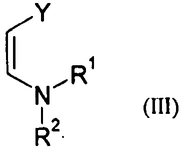

In dieser Formel können
- R¹ und R²: unabhängig voneinander ausgewählt sein aus C₁₋₁₂-Alkyl-, C₃₋₈-Cycloalkyl-, C₂₋₁₂-Alkenyl-, C₂₋₁₂-Alkinyl-, C₆₋₈-Aryl-, C₇₋₁₉-Arylalkyl- oder C₇₋₁₉-Alkylaryl-Gruppen, die jeweils mit einer oder mehreren Gruppen, die ausgewählt sind aus der Gruppe bestehend aus -R', -X, -OR', -SR', -NR'₂, -SiR'₃, -COOR', -(C=O)R', -CN and-CONR₂', wobei R' Wasserstoff oder eine C₁₋₁₂-Alkyl-Gruppe ist, substituiert sein können; oder
- R¹ und R²: zusammen mit dem Stickstoffatom, an das sie gebunden sind, einen 5- bis 6-gliedrigen Ring bilden, der gegebenenfalls ein oder zwei weitere Hetero-Atome, ausgewählt aus O, S und einer SO₂-Gruppe enthalten kann,
- Y: ausgewählt sein aus (C=O)OR³, CN und (C=O)NR⁴R⁵, wobei R³ ,R⁴ und R⁵ unabhängig von einander ausgewählt sind aus C₁₋₁₂-Alkyl-, C₃₋₈-Cycloalkyl, C₂₋₁₂-Alkenyl-, C₂₋₁₂-Alkinyl-, C₆₋₈-Aryl-, C₇₋₁₉-Arylalkyl- oder C₇₋₁₉-Alkylaryl-Gruppen, die jeweils mit einer oder mehreren Gruppen, die ausgewählt sind aus der Gruppe bestehend aus -R', -X, -OR', -SR', -NR'₂, -SiR'₃, -COOR', -(C=O)R', -CN and-CONR₂', wobei R' Wasserstoff oder eine C₁₋₁₂-Alkyl-Gruppe ist, substituiert sein können;

Vorzugsweise können
- R¹ und R²: unabhängig voneinander ausgewählt sein aus Methyl, Ethyl, n-Propyl oder iso-Propyl,
- R¹ und R²: zusammen mit dem Stickstoffatom, an das sie gebunden sind, einen Piperidinyl-, Pyrolidinyl- oder Morpholidinyl-Ring bilden,
- Y: ausgewählt sein aus (C=O)OR³ wobei R³ ausgewählt ist aus Methyl, Ethyl, n-Propyl oder iso-Propyl.

Besonders bevorzugt können
- R¹ und R²: Methyl sein und
- Y: -(C=O)OC₂H₅ sein.

Beispiele für erfindungsgemäß geeignete Dialkylaminoacrylsäureester sind 3-(N,N-Dimethylamino)-acrylsäuremethylester, 3-(N,N-Dimethylamino)-acrylsäureethylester, 3-(N,N-Diethylamino)-acrylsäureethylester, 3-(N,N-Dimethylamino)-acrylsäurenitril, 3-(N,N-Dimethylamino)-acrylsäuredimethylamid und 3-(N,N-Dimethylamino)-acrylsäurediethylamid wobei 3-(N,N-Diethylamino)-acrylsäureethylester besonders bevorzugt ist.

Verfahren zur Herstellung von Dialkylaminoacrylsäureestem sind im Stand der Technik, beispielsweise in EP-A-0 608 725, vorbeschrieben.

Verfahren zur Herstellung von Dialkylaminoacrylnitrilen sind im Stand der Technik, beispielsweise von Rene *et al* in Synthesis (1986), (5), 419-420 beschrieben.

Die Dialkylaminoacrylsäure-Derivate können, falls notwendig, beispielsweise destillativ gereinigt werden. Dies ist im Allgemeinen jedoch im Zusammenhang mit der erfindungsgemäßen Reaktion nicht erforderlich.

Das molare Verhältnis von Dialkylaminoacrylsäure-Derivaten zu eingesetzten fluorierten Carbonsäure kann beispielsweise 0,5 bis 3 betragen, bevorzugt 0,8 bis 2, besonders bevorzugt 1,0 bis 1,5.

### Fluorierte Carbonsäuren (II)

Die gemäß der vorliegenden Erfindung verwendeten fluorierte Carbonsäuren sind Verbindungen der allgemeinen Formel (II) 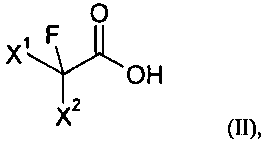 in welcher
- X¹ und X²: unabhängig voneinander für Fluor, Chlor, Brom, Wasserstoff, C₁₋₁₂-Alkylreste, C₁₋₁₂-Haloalkylreste, C₅₋₁₈-Aryl-, C₇₋₁₉-Alkylaryl- oder C₇₋₁₉-Arylalkylreste, vorzugsweise für Wasserstoff, Fluor und Chlor, C₂₋₈-Alkylreste , C₂₋₈-Haloalkylreste, besonders bevorzugt für Fluor, Chlor, Wasserstoff, C₃₋₆-Alkylreste, CF₃ und CF₂H steht.

Beispiele für erfindungsgemäß geeignete fluorierte Carbonäuren sind Trifluoressigsäure, Difluoressigsäure, Difluorchloressigsäure, Chlorfluoressigsäure, 2,3,3,3-Tetrafluorpropionsäure, 2,2,3,3-Tetrafluorpropionsäure, 2,2-Difluorpropionsäure, Pentafluorpropionsäure, 2,3,3,4,4,4-Hexafluorbutancarbonsäure.

### Säurehalogenide (IV)

Die zuvor beschriebenen fluorierten Carbonsäuren werden mit dem Dialkylaminoacrylsäure-Derivat unter Zugabe eines Säurehalogenids der Formel (IV) umgesetzt.

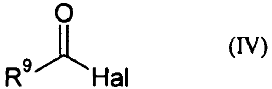

In Formel (IV) ist R⁹ ausgewählt aus C₁₋₁₂-Alkylresten, C₅₋₁₈-Aryl- C₇₋₁₉-Alkylaryl- oder C₇₋₁₉-Arylalkyl- oder Heteroarylresten, vorzugsweise aus C₂₋₈-Alkylresten, besonders bevorzugt aus C₃₋₆-Alkylresten, und Hal ist ausgewählt aus Fluor, Chlor, Brom und Iod, vorzugsweise aus Chlor und Brom, besonders bevorzugt aus Chlor.

Beispiele für erfindungsgemäß geeignete Säurehalogenide sind Essigsäurechlorid, Pivalinsäurechlorid, Isovaleroylchlorid und Benzoylchlorid.

Das molare Verhältnis von Säurehalogeniden der Formel (IV) zur eingesetzten fluorierten Carbonsäure der Formel (II) kann beispielsweise 0,5 bis 5 betragen, bevorzugt 1 bis 3, besonders bevorzugt 2 bis 2,5.

### Basen

Das erfindungsgemäße Verfahren wird in Gegenwart einer Base durchgeführt. Als Basen eignen sich beispielsweise tertiäre Stickstoffbasen, wie z.B, tertiäre Amine, substituierte oder unsubstituierte Pyridine und substituierte oder unsubstituierte Chinoline; Alkali- oder Erdalkalimetallhydroxyde, Hydrogencarbonate oder Carbonate und sonstige anorganische wässrige Basen. Vorzugsweise werden tertiäre Amine, substituierte oder unsubstituierte Pyridine, substituierte oder unsubstituierte Chinoline oder ubstituierte oder unsubstituierte Imidazole und solche der allgemeinen Formel (V) eingesetzt,

NR¹⁰R¹¹R¹² (V),

in welcher
- R¹⁰, R¹¹ und R¹²: unabhängig voneinander für C₁₋₁₂-Alkyl, C₆₋₁₈-Aryl, C₇₋₁₉-Alkylaryl- oder C₇₋₁₉-Arylalkyl stehen können oder jeweils zwei Reste zusammen auch Teil eines 5 bis 8 gliedrigen N-heterocyclischen Restes sein können, oder alle drei Reste zusammen Teil eines N-heterobicyclischen oder N-tricyclischen Restes mit 5 bis 9 Ringatomen pro Cyclus sein können die gegebenenfalls auch andere Heteroatome wie zum Beispiel Sauerstoff enthalten können.

Bevorzugte Beispiele für Basen der allgemeinen Formel (V) sind Triethylamin, Trimethylamin, Diisopropylethylamin, Tri-n-propylamin, Tri-n-butylamin, Tri-n-hexylamin, Tricyclohexylamin, N-Methyl-cyclohexylamin, N-Methylpyrrolidin, N-Methylpiperidin, N-Ethylpiperidin, N,N-Dimethylanilin, N-Methylmorpholin, Pyridin, 2-, 3-, 4-Picolin, 2-Methyl-5-ethyl-pyridin, 2,6-Lutidin, 2,4,6-Collidin, 4-Dimethylaminopyridin, Chinolin, Methylimidazol, Ethylimidazol, Butylimidazol, Chinaldin, N,N,N,N-Tetramethylethylendiamin, N,N-Dimethyl-1,4-diazacyclohexan, N,N-Di-ethyl-1,4-diazacyclohexan, 1,8-Bis-(Dimethylamino)naphthalin, Diazabicylooctan (DABCO), Diazabicyclononan (DBN) und Diazabicycloundecan (DBU). Weiterhin bevorzugt sind Erdalkalimetall- oder Alkalimetallhydroxyde, - carbonate oder- hydrogencarbonate, wie z.B. Natriumhydroxid, Kaliumhydroxid, Lithiumhydroxid, Calciumhydroxid, Natriumcarbonat, Kaliumcarbonat, Natriumhydrogencarbonat und Kaliumhydrogencarbonat eingesetzt. Die anorganische Base kann gegebenenfalls als wässrige Lösung in Konzentrationen zwischen 10 und 40% eingesetzt werden.

Besonders bevorzugt werden Triethylamin, Tributylamin, Methylimidazol, Butylimidazol, Pyridin, 2-, 3-, 4-Picolin, 2-Methyl-5-ethyl-pyridin, 2,6-Lutidin, Natriumhydroxid oder Kaliumhydroxid verwendet.

Das molare Verhältnis von Base zu eingesetzten fluorierten Carbonsäure kann beispielsweise 0,5 bis 10 betragen, bevorzugt 1 bis 8, besonders bevorzugt 1,5 bis 6.

Der Einsatz größerer Mengen an Base ist unkritisch aber unwirtschaftlich.

Bei der Durchführung des erfindungsgemäßen Verfahrens arbeitet man beispielsweise bei Temperaturen von -30 bis 130°C, bevorzugt zwischen 10 und 60°C.

Das erfindungsgemäße Verfahren wird im Allgemeinen unter Normaldruck durchgeführt. Es ist jedoch möglich , unter erhöhtem Druck oder im Vakuum zu arbeiten.

### Lösungsmittel

Die Reaktion kann in Substanz oder in einem Lösungsmittel durchgeführt werden. Vorzugsweise wird die Reaktion in einem Lösungsmittel durchgeführt. Geeignete Lösungsmittel sind beispielsweise ausgewählt aus der Gruppe bestehend aus aliphatischen und aromatischen Kohlenwasserstoffen, wie z.B. n-Hexan, Benzol, Toluol und Xylol, die durch Fluor- und Chloratome substituiert sein können, wie Methylenchlorid, Dichlormethan, Trichlormethan, Tetrachlorkohlenstoff, Fluorbenzol, Chlorbenzol oder Dichlorbenzol; Ethern, wie z.B. Diethylether, Diphenylether Methyl-tert-butylether, Isopropylethylether, Dioxan, Diglym, Dimethylglycol oder THF; Nitrilen wie Methylnitril, Butylnitril oder Phenylnitril; DMF, N,N-Dimethylacetamid wobei Toluol bevorzugt ist.

In einer bevorzugten Ausführungsform des erfindungsgemäßen Verfahrens wird die Base mit der fluorierten Carbonsäure in einem Lösungsmittel oder in Substanz vorgelegt, anschließend wird das Dialkylaminoacrylsäure-Derivat zugegeben und mit dem Carbonsäurechlorid versetzt. Man kann auch die Base mit dem Carbonsäurechlorid in einem Lösungsmittel oder in Substanz vorlegen, anschließend das Dialkylaminoacrylsäure-Derivat zugeben und mit der fluorierten Carbonsäure versetzen.

Bei einer weiteren erfindungsgemäßen Ausführungsform wird die Base mit der fluorierten Carbonsäure in einem Lösungsmittel oder in Substanz vorgelegt, anschließend wird das Carbonsäurechlorid zugegeben und mit dem Dialkylaminoacrylsäure-Derivat versetzt.

Alternativ kann auch die Base das Lösungsmittel ersetzen. Dies ist insbesondere dann zu bevorzugen, wenn auch das Salz der eingesetzten Base unter den vorherrschenden Reaktionsbedingungen flüssig ist.

Zur Aufarbeitung kann man beispielsweise so vorgehen, das man gegebenenfalls ausgefallene Salze zum Beispiel durch Filtration, Zentrifugation oder Sedimentation und Dekantieren abtrennt und die so erhaltene Reaktionslösung entweder direkt weiter umsetzt oder zur Gewinnung der 2-Fluoracyl-3-amino-acrylsäure-Derivate, vorzugsweise bis zur Trockene, einengt. Gegebenenfalls können die erhaltenen 2-Fluoracyl-3-amino-acrylsäure-Derivate auch durch Destillation gereinigt werden. Eine weitere Aufarbeitungsmethode kann durch Zugabe von Wasser zum Reaktionsansatzes und anschließender Phasentrennung erfolgen.

Das erfindungsgemäße Verfahren zum Herstellen von 2-Fluoracyl-3-amino-acrylsäure-Derivaten wird in den nachstehenden Beispielen beschrieben, welche die obige Beschreibung weiter illustrieren. Die Beispiele sind jedoch nicht in einschränkender Weise zu interpretieren.

### Herstellungsbeispiele:

### Beispiel 1

Zu einer Lösung von 32,7 g Triethylamin in 60 ml Toluol werden bei Raumtemperatur zuerst 11,6 g Difluoressigsäure und anschließend 14,35 g Dimethylaminoacrylsäureethylester zugesetzt. Zu dieser Lösung werden 28 g Trimethylacetylchlorid zudosiert und 8 h bei Raumtemperatur gerührt. Die Reaktionsmischung wird auf 30 ml Wasser gegossen, die organische Phase wird abgetrennt, die organische Phase wird mit 20 ml Wasser gewaschen, die vereinigten wässrigen Phasen werden nochmals mit 10 ml Toluol extrahiert. Die vereinigten organischen Phasen werden im Vakuum eingeengt. Man erhält 21 g (94% der Theorie) an 2-(Difluoracetyl)-3-(dimethylamino)-acrylsäureethylester.

### Beispiel 2

Zu einer Lösung von 17,8 g Triethylamin in 50 ml Toluol werden bei Raumtemperatur zuerst 5,7 g Trifluoressigsäure und anschließend 7,23 g Dimethylaminoacrylsäureethylester zugesetzt. Zu dieser Lösung werden 14 g Trimethylacetylchlorid zudosiert und 6 h bei Raumtemperatur gerührt. Die Reaktionsmischung wird auf 20 ml Wasser gegossen, die organische Phase wird abgetrennt, die organische Phase wird mit 20 ml Wasser gewaschen, die vereinigten wässrigen Phasen werden nochmals mit 10 ml Toluol extrahiert. Die vereinigten organischen Phasen werden im Vakuum eingeengt. Man erhält 10,7 g (90 % der Theorie) an 2-(Trifluoracetyl)-3-(dimethylamino)-acrylsäureethylester.

### Beispiel 3

Zu einer Lösung von 17,8 g Triethylamin in 50 ml Toluol werden bei Raumtemperatur zuerst 4,8 g Difluoressigsäure und anschließend 7,23 g Dimethylaminoacrylsäureethylester zugesetzt. Zu dieser Lösung werden 16,3 g Benzoylclorid zudosiert und 6 h bei Raumtemperatur gerührt. Die Reaktionsmischung wird abfiltriert, auf 30 ml Wasser gegossen, die organische Phase wird abgetrennt, die organische Phase wird mit 20 ml Wasser gewaschen, die vereinigten wässrigen Phasen werden nochmals mit 10 ml Toluol extrahiert. Die vereinigten organischen Phasen werden im Vakuum eingeengt. Man erhält 2-(Difluoracetyl)-3-(dimethylamino)-acrylsäureethylester in Ausbeute von 81 %.

### Beispiel 4

Zu einer Lösung von 298 g 1-Methyl-1H-imidazol in 560 ml Toluol werden bei Raumtemperatur (RT) zuerst 97 g Difluoressigsäure und anschließend 144 g Dimethylaminoacrylsäureethylester zugesetzt. Zu dieser Lösung werden 304 g Trimethylacetylchlorid zudosiert und 2 h bei RT gerührt. Nach Reaktionsende werden die Phasen getrennt, die untere Phase einmal mit 50 ml Toluol extrahiert und die vereinigten organischen Phasen im Vakuum eingeengt. Man erhält 2-(Difluoracetyl)-3-(dimethylamino)-acrylsäureethylester in einer Ausbeute von 91%.

## Patentansprüche

1. Verfahren zum Herstellen von 2-Fluoracyl-3-amino-acrylsäure-Derivaten der allgemeinen Formel (I) 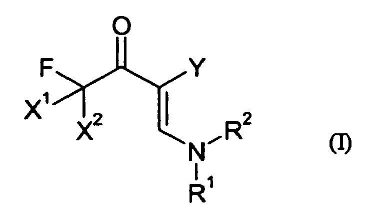 in welcher
R¹ und R² unabhängig voneinander ausgewählt sind aus C₁₋₁₂-Alkyl-, C₃₋₈-Cycloalkyl-, C₂₋₁₂-Alkenyl-, C₂₋₁₂-Alkinyl-, C₆₋₈-Aryl-, C₇₋₁₉-Arylalkyl- oder C₇₋₁₉-Alkylaryl-Gruppen, die jeweils mit einer oder mehreren Gruppen, die ausgewählt sind aus der Gruppe bestehend aus -R', -X, -OR', -SR', -NR'₂, -SiR'₃, -COOR', -(C=O)R', -CN and -CONR₂', wobei R' Wasserstoff oder eine C₁₋₁₂-Alkyl-Gruppe ist, substituiert sein können; oder
R¹ und R² zusammen mit dem Stickstoffatom, an das sie gebunden sind, einen 5- bis 6-gliedrigen Ring bilden können, der gegebenenfalls ein oder zwei weitere Hetero-Atome, ausgewählt aus O, S und einer SO₂-Gruppe, enthalten kann und der mit einer oder mehreren Gruppen, die ausgewählt sind aus der Gruppe bestehend aus -R', -X, -OR', -SR', -NR'₂, -SiR'₃, -COOR', - (C=O)R', -CN and -CONR₂', wobei R' Wasserstoff oder eine C₁₋₁₂-Alkyl-Gruppe ist, substituiert sein kann;
Y ausgewählt ist aus (C=O)OR³, CN und (C=O)NR⁴R⁵, wobei R³ R⁴ und R⁵ unabhängig von einander ausgewählt sind aus C₁₋₁₂-Alkyl-, C₃₋₈-Cycloalkyl, C₂₋₁₂-Alkenyl-, C₂₋₁₂-Alkinyl-, C₆₋₈-Aryl-, C₇₋₁₉-Arylalkyl- oder C₇₋₁₉-Alkylaryl-Gruppen, die jeweils mit einer oder mehreren Gruppen, die ausgewählt sind aus der Gruppe bestehend aus -R', -X, -OR', -SR', -NR'₂, -SiR'₃, -COOR', -(C=O)R', -CN and -CONR₂', wobei R' Wasserstoff oder eine C₁₋₁₂-Alkyl-Gruppe ist, substituiert sein können;
und
X¹ und X² unabhängig voneinander für Fluor, Chlor, Brom, Wasserstoff, C₁₋₁₂-Alkyl-, C₃₋₈-Cycloalkyl-, C₂₋₁₂-Alkenyl-, C₂₋₁₂-Alkinyl-, C₆₋₈-Aryl-, C₇₋₁₉-Arylalkyl- oder C₇₋₁₉-Alkylaryl-Gruppen stehen, wobei die zuvor genannten Alkyl-, Cycloalkyl-, Alkenyl-, Alkinyl-, Aryl-, Arylalkyl- oder Alkylaryl-Gruppen jeweils mit einer oder mehreren Gruppen, die ausgewählt sind aus der Gruppe bestehend aus -R', -X, -OR', -SR', -NR'₂, -SiR'₃, -COOR', -(C=O)R', -CN and -CONR₂', wobei R' Wasserstoff oder eine C₁₋₁₂-Alkyl-Gruppe ist, substituiert sein können;
**dadurch gekennzeichnet, dass** man
fluorierte Carbonsäuren der Formel (II) 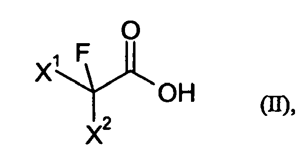 in welcher X¹ und X² die oben genannten Bedeutungen haben, mit 3-Amino-acrylsäure-Derivaten der Formel (III) 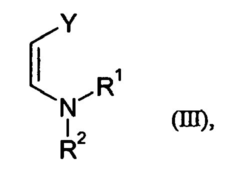 in welcher R¹, R² und Y die oben genannte Bedeutungen haben,
und, in Gegenwart einer Base, mit einem Säurehalogenid der Formel (IV) 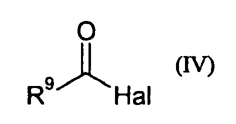 in welcher
R⁹ ausgewählt ist aus C₁₋₁₂-Alkyl-, C₃₋₈-Cycloalkyl-, C₂₋₁₂-Alkenyl-, C₂₋₁₂-Alkinyl-, C₆₋₈-Aryl-, C₇₋₁₉-Arylalkyl- oder C₇₋₁₉-Alkylaryl-Gruppen, die jeweils mit einer oder mehreren Gruppen, die ausgewählt sind aus der Gruppe bestehend aus -R', -X, -OR', -SR', -NR'₂, -SiR'₃, -COOR', - (C=O)R', -CN and -CONR₂', wobei R' Wasserstoff oder eine C₁₋₁₂-Alkyl-Gruppe ist, substituiert sein können;
Hal für Halogen steht; umsetzt.

2. Verfahren zum Herstellen von 2-Fluoracyl-3-amino-acrylsäure-Derivaten der allgemeinen Formel (I) 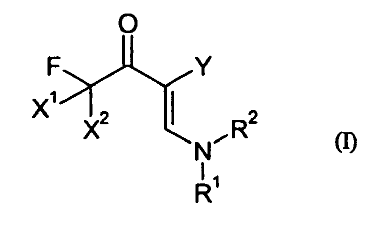 in welcher
R¹ und R² unabhängig voneinander ausgewählt sind aus C₁₋₁₂-Alkylresten, C₅₋₁₈-Aryl-, C₇₋₁₉-Alkylaryl- oder C₇₋₁₉-Arylalkylresten, oder
R¹ und R² zusammen mit dem Stickstoffatom, an das sie gebunden sind, einen 5- bis 6-gliedrigen Ring bilden können, der gegebenenfalls ein oder zwei weitere Hetero-Atome, ausgewählt aus O, S und einer SO₂-Gruppe enthalten kann,
Y ausgewählt ist aus (C=O)OR³, CN und (C=O)NR⁴R⁵, wobei R³ ,R⁴ und R⁵ unabhängig von einander ausgewählt sind aus C₁₋₁₂-Alkylresten, C₅₋₁₈-Aryl-, C₇₋₁₉-Alkylaryl- oder C₇₋₁₉-Arylalkylresten und
X¹ und X² unabhängig voneinander für Fluor, Chlor, Brom, Wasserstoff, C₁₋₁₂-Alkylreste, C₁₋₁₂-Haloalkylreste, C₅₋₁₈-Aryl-, C₇₋₁₉-Alkylaryl- oder C₇₋₁₉-Arylalkylreste stehen,
**dadurch gekennzeichnet, dass** man
fluorierte Carbonsäuren der Formel (II) 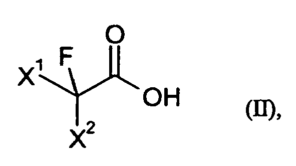 in welcher X¹ und X² die oben genannten Bedeutungen haben, mit 3-Amino-acrylsäure-Derivaten der Formel (III) 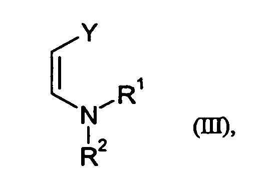 in welcher R¹, R² und Y die oben genannte Bedeutungen haben,
und, in Gegenwart einer Base, mit einem Säurehalogenid der Formel (IV) 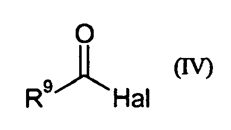 in welcher
R⁹ für C₁₋₁₂-Alkylreste, C₃₋₈-Cycloalkylreste, C₁₋₁₂-Haloalkylreste C₅₋₁₈-Aryl- oder C₇₋₁₉-Arylalkylreste und
Hal für Halogen steht, umsetzt.

3. Verfahren gemäß Anspruch 1 oder 2, wobei
R¹ und R² unabhängig voneinander ausgewählt sind aus Methyl, Ethyl, n-Propyl oder iso-Propyl, oder
R¹ und R² zusammen mit dem Stickstoffatom, an das sie gebunden sind, einen Piperidinyl- oder Pyrolidinyl-Ring bilden;
Y ausgewählt ist aus (C=O)OR³, wobei R³ ausgewählt ist aus Methyl, Ethyl, n-Propyl oder iso-Propyl;
X¹ und X² unabhängig voneinander ausgewählt sind aus Fluor, Chlor, Brom und Wasserstoff;
Hal für Chlor oder Brom steht;
R⁹ ausgewählt ist aus C₁₋₁₂-Alkylresten.

4. Verfahren gemäß irgendeinem der Ansprüche 1 bis 3, wobei
R¹ und R² Methyl sind,
Y (C=O)OC₂H₅;
X¹ Fluor ist;
X² Wasserstoff, ist;
Hal Chlor ist und
R⁹ Methyl ist.

5. Verfahren gemäß irgendeinem der Ansprüche 1 bis 4, wobei die fluorierte Carbonsäuren ausgewählt sind aus der Gruppe bestehend aus Trifluoressigsäure, Difluoressigsäure, Difluorchloressigsäure, Chlorfluoressigsäure, 2,3,3,3-Tetrafluorpropionsäure, 2,2,3,3-Tetrafluorpropionsäure, 2,2-Difluorpropionsäure, Pentafluorpropionsäure, 2,3,3,4,4,4-Hexafluorbutancarbonsäure.

6. Verfahren gemäß irgendeinem der Ansprüche 1 bis 5, wobei die Dialkylaminoacrylsäure-Derivate ausgewählt sind aus der Gruppe bestehend aus 3-(N,N-Dimethylamino)-acrylsäuremethylester, 3-(N,N-Dimethylamino)-acrylsäureethylester, 3-(N,N-Diethylamino)-acrylsäureethylester, 3-(N,N-Dimethylamino)-acrylsäurenitril, 3-(N,N-Dimethylamino)-acrylsäuredimethylamid und 3-(N,N-Dimethylamino)-acrylsäwediethylamid.

7. Verfahren gemäß irgendeinem der Ansprüche 1 bis 6, wobei das molare Verhältnis von Dialkylaminoacrylsäure-Derivaten zu eingesetzten fluorierten Carbonsäure 0,5 bis 3 ist.

8. Verfahren gemäß einem der Ansprüche 1 bis 7, wobei das Säurechlorid ausgewählt ist aus Essigsäurechlorid, Pivalinsäurechlorid, Isovaleroylchlorid und Benzoylchlorid.

9. Verfahren gemäß einem der Ansprüche 1 bis 8, wobei das molare Verhältnis von Säurechloride zur eingesetzten fluorierten Carbonsäure 0,5 bis 5 ist.

10. Verfahren gemäß einem der Ansprüche 1 bis 9, wobei die Base ausgewählt ist aus Triethylamin, Trimethylamin, Düsopropylethylamin, Tri-n-propylamin, Tri-n-butylamin, Tri-n-hexylamin, Tricyclohexylamin, N-Methyl-cyclohexylamin, N-Methylpyrrolidin, N-Methylpiperidin, N-Ethylpiperidin, N,N-Dimethylanilin, N-Methylmorpholin, Pyridin, 2-, 3-, 4-Picolin, 2-Methyl-5-ethyl-pyridin, 2,6-Lutidin, 2,4,6-Collidin, 4-Dimethylaminopyridin, Chinolin, Methylimidazol, Ethylimidazol, Butylimidazol, Chinaldin, N,N,N,N-Tetramethylethylendiamin, N,N-Dimethyl-1,4-diazacyclohexan, N,N-Di-ethyl-1,4-diazacyclohexan, 1,8-Bis-(Dimethylamino)naphthalin, Diazabicylooctan (DABCO), Diazabicyclononan (DBN) und Diazabicycloundecan (DBU), Natriumhydroxid, Kaliumhydroxid, Lithiumhydroxid, Calciumhydroxid, Natriumcarbonat, Kaliumcarbonat, Natriumhydrogencarbonat und Kaliumhydrogencarbonat.

## Claims

1. Process for preparing 2-fluoroacyl-3-aminoacrylic acid derivatives of the general formula (I) 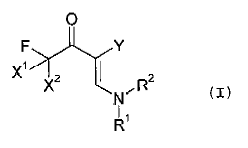 in which
R¹ and R² are each independently selected from C₁₋₁₂-alkyl, C₃₋₈-cycloalkyl, C₂₋₁₂-alkenyl, C₁₋₁₂-alkynyl, C₆₋₈-aryl, C₇₋₁₉-arylalkyl and C₇₋₁₉-alkylaryl groups, each of which may be substituted by one or more groups selected from the group consisting of - R', -X, -OR', -SR', -NR'₂, -SiR'₃, -COOR', -(C=O)R', -CN and -CONR₂', where R' is hydrogen or a C₁₋₁₂-alkyl group; or
R¹ and R², together with the nitrogen atom to which they are bonded, may form a 5- to 6-membered ring which may optionally contain one or two further heteroatoms selected from O, S and an SO₂ group, and which may be substituted by one or more groups selected from the group consisting of -R', -X, -OR', -SR', -NR'₂, -SiR'₃, -COOR', -(C=O)R', -CN and -CONR₂', where R' is hydrogen or a C₁₋₁₂-alkyl group;
Y is selected from (C=O)OR³, CN and (C=O)NR⁴R⁵, Where R³ R⁴ and R⁵ are each independently selected from C₁₋₁₂-alkyl, C₃₋₈-cycloalkyl, C₂₋₁₂-alkenyl, C₂₋₁₂-alkynyl, C₆₋₈-aryl, C₇₋₁₉-arylalkyl- and C₇₋₁₉-alkylaryl groups, each of which may be substituted by one or more groups selected from the group consisting of-R', -X, -OR', -SR', -NR'₂, -SiR'₃, -COOR', -(C=O)R', -CN and -CONR₂', where R' is hydrogen or a C₁₋₁₂-alkyl group;
and
X¹ and X² are each independently fluorine, chlorine, bromine, hydrogen, C₁₋₁₂-alkyl, C₃₋₈-cycloalkyl, C₂₋₁₂-alkenyl, C₂₋₁₂-alkynyl, C₆₋₈-aryl, C₇₋₁₉-arylalkyl or C₇₋₁₉-alkylaryl groups, where the aforementioned alkyl, cycloalkyl, alkenyl, alkynyl, aryl, arylalkyl and alkylaryl groups may each be substituted by one or more groups selected from the group consisting of -R', -x, -OR', -SR', -NR'₂, -SiR'₃, -COOR', -(C=O)R', -CN and -CONR₂', where R' is hydrogen or a C₁₋₁₂-alkyl group;
**characterized in that**
fluorinated carboxylic acids of the formula (II) 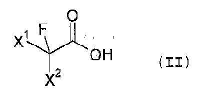 in which X¹ and X² are each as defined above are reacted with 3-aminoacrylic acid derivatives of the formula (III) 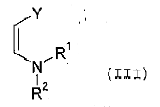 in which R¹, R² and Y are each as defined above,
and, in the presence of a base, with an acid halide of the formula (IV) 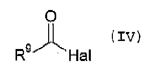 in which
R⁹ is selected from C₁₋₁₂-alkyl, C₃₋₈-cycloalkyl, C₂₋₁₂-alkenyl, C₂₋₁₂-alkynyl, C₆₋₈-aryl, C₇₋₁₉-arylalkyl- or C₇₋₁₉-alkylaryl groups, each of which may be substituted by one or more groups selected from the group consisting of -R', -X, -OR", -SR', -NR'₂, -SiR'₃, -COOR', -(C=O)R', -CN and -CONR₂', where R' is hydrogen or a C₁₋₁₂-alkyl group;
Hal is halogen.

2. Process for preparing 2-fluoroacyl-3-aminoacrylic acid derivatives of the general formula (I) 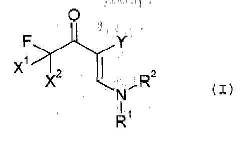 in which
R¹ and R² are each independently selected from C₁₋₁₂-alkyl radicals, C₅₋₁₈-aryl, C₇₋₁₉-alkylaryl or C₇₋₁₉-arylalkyl radicals, or
R¹ and R², together with the nitrogen atom to which they are bonded, may form a 5- to 6-membered ring which may optionally contain one or two further heteroatoms selected from O, S and an SO₂ group,
Y is selected from (C=O)OR³, CN and (C=O)NR⁴R⁵, where R³, R⁴ and R⁵ are each independently selected from C₁₋₁₂-alkyl radicals, C₅₋₁₈-aryl, C₇₋₁₉-alkylaryl and C₇₋₁₉-arylalkyl radicals and
X¹ and X² are each independently fluorine, chlorine, bromine, hydrogen, C₁₋₁₂-alkyl radicals, C₁₋₁₂-haloalkyl radicals, C₅₋₁₈-aryl, C₇₋₁₉-alkylaryl or C₇₋₁₉-arylalkyl radicals,
**characterized in that**
fluorinated carboxylic acids of the formula (II) 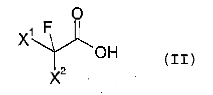 in which X¹ and X² are each as defined above are reacted with 3-aminoacrylic acid derivatives of the formula (III) 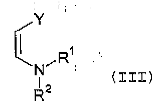 in which R¹, R² and Y are each as defined above,
and, in the presence of a base, with an acid halide of the formula (IV) 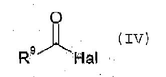 in which
R⁹ is C₁₋₁₂-alkyl radicals, C₃₋₈-cycloalkyl radicals, C₁₋₁₂-haloalkyl radicals, C₅₋₁₈-aryl or C₇₋₁₉-arylalkyl radicals and
Hal is Halogen.

3. Process according to Claim 1 or 2, wherein
R¹ and R² are each independently selected from methyl, ethyl, n-propyl and isopropyl, or
R¹ and R², together with the nitrogen atom to which they are bonded, form a piperidinyl or pyrrolidinyl ring;
Y is selected from (C=O)OR³ where R³ is selected from methyl, ethyl, n-propyl and isopropyl;
X¹ and X² are each independently selected from fluorine, chlorine, bromine and hydrogen;
Hal is chlorine or bromine;
R⁹ is selected from C₁₋₁₂-alkyl radicals.

4. Process according to any one of Claims 1 to 3, wherein
R¹ and R² are each methyl,
Y is (C=O)OC₂H₅;
X¹ is fluorine;
X² is hydrogen;
Hal is chlorine and
R⁹ is methyl.

5. Process according to any one of Claims 1 to 4, wherein the fluorinated carboxylic acids are selected from the group consisting of trifluoroacetic acid, difluoroacetic acid, difluorochloroacetic acid, chlorofluoroacetic acid, 2,3,3,3-tetrafluoropropionic acid, 2,2,3,3-tetrafluoropropionic acid, 2,2-difluoropropionic acid, pentafluoropropionic acid, 2,3,3,4,4,4-hexafluorobutanecarboxylic acid.

6. Process according to any one of Claims 1 to 5, wherein the dialkylaminoacrylic acid derivatives are selected from the group consisting of methyl 3-(N,N-dimethylamino)-acrylate, ethyl 3-(N,N-dimethylamino)acrylate, ethyl 3-(N,N-diethylamino)acrylate, 3-(N,N-dimethylamino)acrylonitrile, N,N-dimethyl-3-(N,N-dimethylamino)acrylamide and N,N-diethyl-3-(N,N-dimethylamino) acrylamide.

7. Process according to any one of Claims 1 to 6, wherein the molar ratio of dialkylaminoacrylic acid derivatives to fluorinated carboxylic acid used is 0.5 to 3.

8. Process according to any of Claims 1 to 7, wherein the acid chloride is selected from acetyl chloride, pivaloyl chloride, isovaleroyl chloride and benzoyl chloride.

9. Process according to any of Claims 1 to 8, wherein the molar ratio of acid chloride to the fluorinated carboxylic acid used is 0.5 to 5.

10. Process according to any of Claims 1 to 9, wherein the base is selected from triethylamine, trimethylamine, diisopropylethylamine, tri-n-propylamine, tri-n-butylamine, tri-n-hexylamine, tricyclohexylamine, N-methylcyclohexylamine, N-methylpyrrolidine, N-methylpiperidine, N-ethylpiperidine, N,N-dimethylaniline, N-methylmorpholine, pyridine, 2-, 3-, 4-picoline, 2-methyl-5-ethylpyridine, 2,6-lutidine, 2,4,6-collidine, 4-dimethylaminopyridine, quinoline, methylimidazole, ethylimidazole, butylimidazole, quinaldine, N,N,N,N-tetramethylethylenediamine, N,N-dimethyl-1,4-diazacyclohexane, N,N-diethyl-1,4-diazacyclohexane, 1,8-bis(dimethylamino)naphthalene, diazabicylooctane (DABCO), diazabicyclononane (DBN) and diazabicycloundecane (DBU), sodium hydroxide, potassium hydroxide, lithium hydroxide, calcium hydroxide, sodium carbonate, potassium carbonate, sodium hydrogencarbonate and potassium hydrogencarbonate.

## Revendications

1. Procédé pour la préparation de dérivés de l'acide 2-fluoroacyl-3-aminoacrylique de formule générale (I) 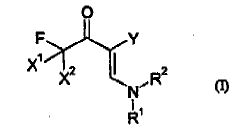 dans laquelle
R¹ et R² sont choisis, indépendamment l'un de l'autre, parmi les groupes C₁₋₁₂-alkyle, C₃₋₈-cycloalkyle, C₂₋₁₂-alcényle, C₂₋₁₂-alcynyle, C₆₋₈-aryle, C₇₋₁₉-arylalkyle ou C₇₋₁₉-alkylaryle, qui peuvent à chaque fois être substitués par un ou plusieurs groupes choisis dans le groupe constitué par -R', -X, -OR', -SR', -NR'₂, -SiR'₃, -COOR', -(C=O)R', -CN et -CONR₂', R' représentant hydrogène ou un groupe C₁₋₁₂-alkyle ; ou
R¹ et R² représentent, ensemble avec l'atome d'azote auquel ils sont liés, un cycle de 5 à 6 chaînons, qui peut contenir le cas échéant un ou deux autres hétéroatomes, choisis parmi O, S et un groupe SO₂, et qui peut être substitué par un ou plusieurs groupes choisis dans le groupe constitué par -R', -X, -OR', -SR', -NR'₂, -SiR'₃, -COOR', - (C=O)R', -CN et -CONR₂', R' représentant hydrogène ou un groupe C₁₋₁₂-alkyle ;
Y est choisi parmi (C=O)OR³, CN et (C=O)NR⁴R⁵, R³ ,R⁴ et R⁵ étant choisis, indépendamment les uns des autres, parmi les groupes C₁₋₁₂-alkyle, C₃₋₈-cycloalkyle, C₂₋₁₂-alcényle, C₂₋₁₂-alcynyle, C₆₋₈-aryle, C₇₋₁₉-arylalkyle ou C₇₋₁₉-alkylaryle, qui peuvent à chaque fois être substitués par un ou plusieurs groupes qui sont choisis dans le groupe constitué par -R', -X, -OR', -SR', -NR'₂, -SiR'₃, -COOR', -(C=O)R', -CN et -CONR₂', R' représentant hydrogène ou un groupe C₁₋₁₂-alkyle ; et
X¹ et X² représentent, indépendamment l'un de l'autre, fluor, chlore, brome, hydrogène, des groupes C₁₋₁₂-alkyle, C₃₋₈-cycloalkyle, C₂₋₁₂-alcényle, C₂₋₁₂-alcynyle, C₆₋₈-aryle, C₇₋₁₉-arylalkyle ou C₇₋₁₉-alkylaryle, les groupes alkyle, cycloalkyle, alcényle, alcynyle, aryle, arylalkyle ou alkylaryle susmentionnés pouvant à chaque fois être substitués par un ou plusieurs groupes qui sont choisis dans le groupe constitué par -R', -X, -OR', -SR', -NR'₂, ₋SiR'₃, -COOR', -(C=O)R', -CN et -CONR₂', R' représentant hydrogène ou un groupe C₁₋₁₂-alkyle ;
**caractérisé en ce qu'**on transforme des acides carboxyliques fluorés de formule (II) 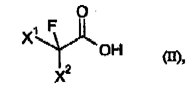 dans laquelle X¹ et X² présentent les significations susmentionnées, avec des dérivés de l'acide 3-aminoacrylique de formule (III) 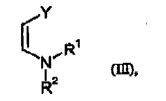 dans laquelle R¹, R² et Y ont les significations susmentionnées,
et, en présence d'une base, avec un halogénure d'acide de formule (IV) 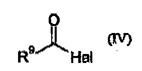 dans laquelle
R⁹ est choisi parmi les groupes C₁₋₁₂-alkyle, C₃₋₈-cycloalkyle, C₂₋₁₂-alcényle, C₂₋₁₂-alcynyle, C₆₋₈-aryle, C₇₋₁₉-arylalkyle ou C₇₋₁₉-alkylaryle, qui peuvent à chaque fois être substitués par un ou plusieurs groupes choisis dans le groupe constitué par -R', -X, -OR', -SR', -NR'₂, -SiR'₃, -COOR', -(C=O)R', -CN et -CONR₂', R' représentant hydrogène ou un groupe C₁₋₁₂-alkyle ;
Hal représente halogène.

2. Procédé pour la préparation de dérivés de l'acide 2-fluoroacyl-3-aminoacrylique de formule générale (I) 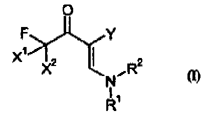 dans laquelle
R¹ et R² sont choisis, indépendamment l'un de l'autre, parmi les radicaux C₁₋₁₂-alkyle, C₅₋₁₈-aryle, C₇₋₁₉-alkylaryle ou C₇₋₁₉-arylalkyle, ou
R¹ et R² représentent, ensemble avec l'atome d'azote auquel ils sont liés, un cycle de 5 à 6 chaînons, qui peut contenir le cas échéant un ou deux autres hétéroatomes, choisis parmi 0, S et un groupe SO₂,
Y est choisi parmi (C=O)OR³, CN et (C=O)NR⁴R³, R³ R⁴ et R⁵ étant choisis indépendamment les uns des autres parmi les radicaux C₁₋₁₂-alkyle, C₅₋₁₈-aryle, C₇₋₁₉-alkylaryle ou C₇₋₁₉-arylalkyle et
X¹ et X² représentent, indépendamment l'un de l'autre fluor, chlore, brome, hydrogène, des radicaux C₁₋₁₂-alkyle, C₁₋₁₂-halogénoalkyle, C₅₋₁₈-aryle, C₇₋₁₉-alkylaryle ou C₇₋₁₉-arylalkyle,
**caractérisé en ce qu'**on transforme des acides carboxyliques fluorés de formule (II) 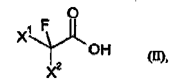 dans laquelle X¹ et X² présentent les significations susmentionnées, avec des dérivés de l'acide 3-aminoacrylique de formule (III) 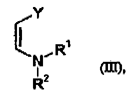 dans laquelle R¹, R² et Y ont les significations susmentionnées,
et, en présence d'une base, avec un halogénure d'acide de formule (IV) 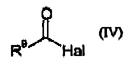 dans laquelle
R⁹ représente des radicaux C₁₋₁₀-alkyle, C₃₋₈-cycloalkyle, C₁₋₁₂-halogénoalkyle, C₅₋₁₈-aryle ou C₇₋₁₉-arylalkyle et
Hal représente halogène.

3. procédé selon la revendication 1 ou 2,
R¹ et R² étant choisis, indépendamment l'un de l'autre, parmi méthyle, éthyle, n-propyle ou iso-propyle, ou
R¹ et R² formant ensemble avec l'atome d'azote auquel ils sont liés un radical pipéridinyle ou pyrrolidinyle ;
Y étant choisi parmi (C=O)OR³, R³ étant choisi parmi méthyle, éthyle, n-propyle ou iso-propyle ;
X¹ et X² étant choisis, indépendamment l'un de l'autre, parmi fluor, chlore, brome et hydrogène ;
Hal représentant chlore ou brome ;
R⁹ étant choisi parmi les radicaux C₁₋₁₂-alkyle.

4. Procédé selon l'une quelconque des revendications 1 à 3,
R¹ et R² représentant méthyle,
Y représentant (C=O) OC₂H₅ ;
X¹ représentant fluor ;
X² représentant hydrogène ;
Hal représentant chlore et
R⁹ représentant méthyle.

5. Procédé selon l'une quelconque des revendications 1 à 4, les acides carboxyliques fluorés étant choisis dans le groupe constitué par l'acide trifluoroacétique, l'acide difluoroacétique, l'acide difluorochloroacétique, l'acide chlorofluoroacétique, l'acide 2,3,3,3-tétrafluoropropionique, l'acide 2,2,3,3-tétrafluoropropionique, l'acide 2,2-difluoropropionique, l'acide pentafluoropropionique, l'acide 2,3,3,4,4,4-hexafluorobutanecarboxylique.

6. Procédé selon l'une quelconque des revendications 1 à 5, les dérivés de l'acide dialkylaminoacrylique étant choisis dans le groupe constitué par l'ester méthylique de l'acide 3-(N,N-diméthylamino)-acrylique, l'ester éthylique de l'acide 3-(N,N-diméthylamino)-acrylique, l'ester éthylique de l'acide 3-(N,N-diéthylamino)-acrylique, le nitrile de l'acide 3-(N,N-diméthylamino)-acrylique, le diméthylamide de l'acide 3-(N,N-diméthylamino)-acrylique et le diéthylamide de l'acide 3-(N,N-diméthylamino)-acrylique.

7. Procédé selon l'une quelconque des revendications 1 à 6, le rapport molaire des dérivés d'acide dialkylaminoacrylique à l'acide carboxylique fluoré utilisé valant 0,5:3.

8. Procédé selon l'une quelconque des revendications 1 à 7, le chlorure d'acide étant choisi parmi le chlorure de l'acide acétique, le chlorure de l'acide pivalique, le chlorure d'isovaléroyle et le chlorure de benzoyle.

9. Procédé selon l'une quelconque des revendications 1 à 8, le rapport molaire de chlorure d'acide à l'acide carboxylique fluoré utilisé valant 0,5:5.

10. Procédé selon l'une quelconque des revendications 1 à 9, la base étant choisie parmi la triéthylamine, la triméthylamine, la diisopropyléthylamine, la tri-n-propylamine, la tri-n-butylamine, la tri-n-hexylamine, la tricyclohexylamine, la N-méthylcyclohexylamine, la N-méthylpyrrolidine, la N-méthylpipéridine, la N-éthylpipéridine, la N,N-diméthylaniline, la N-méthylmorpholine, la pyridine, la 3-picoline, la 3-picoline, la 4-picoline, la 2-méthyl-5-éthylpyridine, la 2,6-lutidine, la 2,4,6-collidine, la 4-diméthylaminopyridine, la quinoléine, le méthylimidazole, l'éthylimidazole, le butylimidazole, la quinaldine, la N,N,N,N-tétraméthyléthylènediamine, le N,N-diméthyl-1,4-diazacyclohexane, le N,N-diéthyl-1,4-diazacyclohexane, le 1,8-bis-(diméthylamino)naphtalène, le diazabicyclooctane (DABCO), le diazabicyclononane (DBN) et le diazabicyclo-undécane (DBU), l'hydroxyde de sodium, l'hydroxyde de potassium, l'hydroxyde de lithium, l'hydroxyde de calcium, le carbonate de sodium, le carbonate de potassium, l'hydrogénocarbonate de sodium et l'hydrogénocarbonate de potassium.
